# EUROPÄISCHE PATENTANMELDUNG

(11) **EP 1 816 472 A1**
(43) Veröffentlichungstag der Anmeldung: **08.08.2007**
(21) Anmeldenummer: 06002308.2
(22) Anmeldetag: 03.02.2006
(51) Int. Cl.: G01N 27/72

(54) **Vorrichtung und Verfahren zur Bestimmung der Dichte magnetisierbarer Partikel in einem pastösen Material**

(71) Anmelder: Bakker Holding Son B.V., 5692 EL Son (NL)
(72) Erfinder: Zhang, Shunli, 5583 GK Waalre (NL)
(74) Vertreter: Cohausz & Florack

(57) **Zusammenfassung**

Die Erfindung betrifft eine Vorrichtung zur Bestimmung der Dichte magnetisierbarer Partikel in einem pastösen Material (5) mit einem Flächenabschnitt (1), entlang dessen das pastöse (5) Material fließt und mit wenigstens einer ein Magnetfeld erzeugenden Magneteinheit (2), wobei das Magnetfeld der wenigstens einen Magneteinheit (2) in einem ersten Arbeitszustand der Vorrichtung eine anziehende Kraft auf die magnetisierbaren Partikel (5*) in dem entlang des Flächenabschnitts (1) fließenden pastösen Material (5) derart ausübt, dass die in dem pastösen Material (5) enthaltenen magnetisierbaren Partikel (5*) aus dem pastösen Material (5) entfernt werden, und wobei das Magnetfeld der wenigstens einen Magneteinheit (2) in einem zweiten Arbeitszustand der Vorrichtung die magnetisierbaren Partikel (5*) wieder freigibt. Ferner betrifft die Erfindung ein Verfahren zur Bestimmung der Dichte magnetisierbarer Partikel in einem pastösen Material (5) unter Verwendung der genannten Vorrichtung.

## Beschreibung

Die Erfindung betrifft eine Vorrichtung und ein Verfahren zur Bestimmung der Dichte magnetisierbarer Partikel in einem pastösen Material.

Die Verwendung von Stahlfasern in Beton zu dessen Verstärkung ist seit über 20 Jahren bekannt. Stahlfaserverstärkter Beton zeigt eine Reihe von Vorteilen gegenüber nichtverstärktem Beton, so z.B. eine Verbesserung des Verhaltens unter Biegebelastung oder des Rissverhaltens. Neben den erwähnten Fasern finden - je nach Anwendungshintergrund - auch andere Geometrien, beispielsweise Stahlringe Verwendung.

Für die Bereitstellung eines stahlfaserverstärkten Betons mit spezifizierten Eigenschaften ist es von entscheidender Bedeutung, dass die Dichte der Fasern in einer Betonmischung genau bestimmbar ist.

Es ist daher Aufgabe der Erfindung, eine Vorrichtung zu schaffen, die einfach aufgebaut ist, zuverlässig arbeitet und sich zudem in der Praxis als robust erweist. Ferner ist es Aufgabe der Erfindung, ein einfach durchzuführendes Verfahren zur Bestimmung der Dichte magnetisierbarer Partikel in einem pastösen Material anzugeben.

Die Aufgabe wird erfindungsgemäß gelöst mit einer Vorrichtung mit einem Flächenabschnitt, entlang dessen das pastöse Material fließt, und mit wenigstens einer ein Magnetfeld erzeugenden Magneteinheit, wobei das Magnetfeld der wenigstens einen Magneteinheit in einem ersten Arbeitszustand der Vorrichtung eine anziehende Kraft auf die magnetisierbaren Partikel in dem entlang des Flächenabschnitts fließenden pastösen Material derart ausübt, dass die in dem pastösen Material enthaltenen magnetisierbaren Partikel aus dem pastösen Material entfernt werden, und wobei das Magnetfeld der wenigstens einen Magneteinheit in einem zweiten Arbeitszustand der Vorrichtung die magnetisierbaren Partikel wieder freigibt.

Die erfindungsgemäße Vorrichtung umfasst lediglich zwei Grundkomponenten, nämlich den Flächenabschnitt sowie die Magneteinheit, und ist somit sehr einfach aufgebaut und entsprechend robust in der Anwendung. Die Vorrichtung wird in zwei Arbeitszuständen betrieben, wobei die magnetisierbaren Partikel im ersten Arbeitszustand der Vorrichtung in der beschriebenen Weise aus dem pastösen Material magnetisch herausgelöst werden, während sie im zweiten Arbeitszustand der Vorrichtung wieder freigegeben werden und somit für quantitative Messungen, beispielsweise für eine Bestimmung ihres Gesamtgewichtes zur Verfügung stehen. Zudem können auch die Form der Partikel und insbesondere nach dem Mischen mit dem pastösen Material eventuell erfolgte Verformungen durch Sichten der aus dem pastösen Material magnetisch herausgelösten Partikel festgestellt werden. Ferner zeichnet sich die Vorrichtung durch ein hohes Maß an Genauigkeit aus, da annähernd alle in dem pastösen Material vorhandenen Partikel erfasst werden. Dabei kann das jeweils untersuchte pastöse Material eine beinahe beliebige Viskosität aufweisen. Ebenso ist die Partikeldichte in einem weiten Bereich variierbar, ohne dass dies einen Einfluss auf das Messergebnis hat. Insgesamt lassen sich mit der Vorrichtung exakte und reproduzierbare Messungen in kurzer Zeit ausführen. Vorzugsweise weist der Flächenabschnitt ein Gefälle auf, so dass das pastöse Material mit konstanter Geschwindigkeit ohne zusätzliche Hilfsmittel entlang des Flächenabschnitts fließen kann.

In einer sehr einfachen Ausführung ist der Flächenabschnitt beispielsweise als geneigte Fläche ausgebildet, über der die Magneteinheit aufgehängt ist. Das pastöse, die magnetisierbaren Partikel enthaltende Material wird an dem oberen Ende der Fläche aufgegeben und fließt entlang der Fläche unterhalb der Magneteinheit abwärts, wobei die magnetisierbaren Partikel durch die Magneteinheit angezogen und entsprechend nach oben aus dem pastösen Material herausgelöst werden.

Nach einer ersten vorteilhaften Ausgestaltung der Erfindung ist der Flächenabschnitt als Kanalabschnitt mit einer Seitenwandung sowie einer stirnseitigen Eintrittsöffnung und einer stirnseitigen Austrittsöffnung ausgebildet. Die Breite des Kanalabschnitts, d.h. der Abstand der seitlichen Führungsflächen, ist dabei auf die räumliche Feldverteilung der Magneteinheit abgestimmt, so dass über die Breite des Kanalabschnitts sämtliche Partikel aus dem pastösen Material herausgelöst werden können. Ein solcher Kanalabschnitt kann auf vielfältige Weise gestaltet sein. So kann die Seitenwandung des Kanalabschnitts einseitig offen sein, so dass das pastöse Material, während es durch den Kanalabschnitt fließt, einseitig zugänglich ist. Nach einer bevorzugten Ausgestaltung der Erfindung weist der Kanalabschnitt eine umfangsmäßig geschlossene Seitenwandung auf. Bei einem beispielsweise rechteckigen, insbesondere quadratischen Querschnitt des Kanalabschnitts ist dieser folglich als Vierkantrohr ausgebildet. Ebenso kann der Querschnitt auch rund, insbesondere kreisrund ausgebildet sein.

Der Kanalabschnitt kann ferner auf unterschiedliche Weise positioniert sein. So ist es möglich, den Kanalabschnitt horizontal oder geneigt zu positionieren. Hierbei ist es möglich den Kanalabschnitt mit einseitig offener Wandung auszubilden. Im Falle eines horizontal angeordneten Kanalabschnitts muss das pastöse Material mittels einer Pumpe oder einer der Eintrittsöffnung des Kanalabschnittes vorgeordneten Gefällestrecke jedoch zunächst beschleunigt werden, da der Kanalabschnitt dann selbst kein Gefälle aufweist. Nach einer besonders bevorzugten Ausgestaltung der Erfindung ist der Kanalabschnitt im wesentlichen vertikal mit oben liegender Eintrittsöffnung angeordnet, wobei in diesem Fall sinnvollerweise nur ein Kanalabschnitt mit umfangsmäßig geschlossener Wandung zum Einsatz kommen kann. Der besondere Vorteil eines im wesentlichen vertikal angeordneten Kanalabschnittes liegt darin, dass hier ein maximiertes Gefälle vorliegt, weshalb es sich auch für besonders viskose und damit nur schlecht fließfähige pastöse Materialien eignet.

Ferner kann der Kanalabschnitt auch hinsichtlich seiner Längserstreckung unterschiedlich ausgeformt sein. So weist der Kanalabschnitt nach einer Ausgestaltung der Erfindung eine über die Längserstreckung des Kanalabschnitts konstante Querschnittsfläche auf. Ferner kann sich die Querschnittsfläche des Kanalabschnitts nach einer alternativen Ausgestaltung der Erfindung entlang der Längserstreckung des Kanalabschnitts verändern, insbesondere von der Eintrittsöffnung des Kanalabschnitts in Richtung der Austrittsöffnung des Kanalabschnitts verringern, so dass der Kanalabschnitt insgesamt in Richtung seiner Austrittsöffnung konisch zulaufend ausgebildet ist. Entsprechend kann die Eintrittsöffnung des Kanalabschnitts mit besonders großer Querschnittsfläche ausgebildet sein, was das Einfüllen des pastösen Materials erleichtert. Alternativ oder ergänzend kann die Eintrittsöffnung mit einem Einfülltrichter versehen sein.

Um vergleichbare Ergebnisse zu erhalten, ist es sinnvoll, die Vorrichtung in standardisierter Weise zu verwenden. Insbesondere ist es zweckmäßig, stets gleiche Volumina pastösen Materials durch den Kanalabschnitt fließen zu lassen. Hierzu ist nach einer weiteren vorteilhaften Ausgestaltung der Erfindung vorgesehen, dass der Eintrittsöffnung in Flussrichtung des pastösen Materials ein Speichervolumen für das pastöse Material vorgeordnet ist. Bei der Durchführung einer standardisierten Dichtemessung wird zunächst das Speichervolumen vollständig oder bis zu einer Eichmarkierung befüllt und anschließend der Inhalt des Speichervolumens in den Kanalabschnitt eingeleitet. Hierzu ist die Eintrittsöffnung vorzugsweise durch eine variable Verschlusseinrichtung verschließbar.

Neben dem Flächenabschnitt kann auch die Magneteinheit auf verschiedene Weise gestaltet sein. So kann die Magneteinheit mit einem oder mehreren Elektromagneten und/oder einem oder mehreren Permanentmagneten versehen sein. Im Falle eines Elektromagneten ist dieser im ersten Arbeitszustand der Vorrichtung eingeschaltet, so dass er ein auf den Flächenabschnitt einwirkendes magnetisches Feld erzeugt, und im zweiten Arbeitszustand der Vorrichtung ausgeschaltet.

Permanentmagneten erzeugen im Gegensatz zu Elektromagneten ein zeitlich nicht veränderliches Magnetfeld. Um die magnetisierbaren Partikel im ersten Arbeitszustand der Vorrichtung mit einer anziehenden Kraft zu beaufschlagen und im zweiten Arbeitszustand wieder freizugeben, ist nach einer besonders bevorzugten Ausgestaltung der Erfindung daher vorgesehen, dass die Magneteinheit wenigstens einen Permanentmagneten umfasst, welcher relativ zu dem Flächenabschnitt aus einer ersten Position, in der sich die Vorrichtung im ersten Arbeitszustand befindet, in eine zweite Position, in der sich die Vorrichtung im zweiten Arbeitszustand befindet, und in umgekehrter Richtung bewegbar ist. Die Art der Bewegung ist dabei nicht festgelegt und kann gemäß der Lehre der Erfindung auf verschiedene Weise realisiert sein. So kann die Magneteinheit mit dem Flächenabschnitt gelenkig verbunden sein, wobei die Magneteinheit durch eine Schwenkbewegung nach Art einer Tür aus der zweiten Position in die erste Position gebracht wird, in welcher das Magnetfeld des Permanentmagneten derart räumlich in den Kanalabschnitt einwirkt, dass die magnetisierbaren Partikel des entlang des Kanalabschnitts fließenden pastösen Materials mit einer anziehenden Kraft beaufschlagt werden. Ebenso ist auch eine rein translatorische Bewegung möglich, in der die Magneteinheit durch eine geradlinige Bewegung an den Flächenabschnitt angenähert wird.

In beiden beschriebenen Fällen ist es aus praktischen Gründen zweckmäßig, die Magneteinheit in der ersten Position mit dem Flächenabschnitt durch Klemmen zu verbinden.

Nach einer weiteren zweckmäßigen Ausgestaltung der Erfindung ist vorgesehen, dass die Vorrichtung eine mit dem Flächenabschnitt fest verbundene Aufnahmeeinrichtung umfasst, in welche der wenigstens eine Permanentmagnet einführbar ist. Vorzugsweise ist die Aufnahmevorrichtung mit dem Flächenabschnitt permanent verbunden. Hierdurch wird erreicht, dass lediglich der Permanentmagnet zur Einstellung des jeweiligen Arbeitszustandes der Vorrichtung bewegt werden muss, während weitere in bezug auf die Funktion der Magneteinheit nicht wirksame mechanische Komponenten, wie Adapterstücke u.ä., fest mit dem Flächenabschnitt verbunden bleiben können.

Die Magneteinheit kann nach der Lehre der Erfindung ein Magnetfeld mit verschiedenartiger Feldgeometrie erzeugen. Als besonders geeignet erweisen sich von einer Multipolanordnung erzeugte Magnetfelder. Diese haben den Vorteil, eine starke Haltekraft auf magnetisierbare Partikel auszuüben, so dass diese Partikel durch den sich entlang des Flächenabschnitts fortbewegenden Materialstrom nicht wieder mitgerissen werden und gleichzeitig der Materialstrom durch die an der Seitenwand anhaftenden Partikel nicht behindert wird. Insbesondere kann die Multipolanordnung als Halbach-Array ausgebildet sein.

Die der Erfindung zugrunde liegende Aufgabe wird ferner gelöst durch ein Verfahren zur Bestimmung der Dichte magnetisierbarer Partikel in einem pastösen Material unter Verwendung einer Vorrichtung gemäß den Ansprüchen 1 bis 19, welches durch die folgenden Verfahrensschritte gekennzeichnet ist:
- die Vorrichtung wird in den ersten Arbeitszustand gebracht
- ein definiertes Volumen des pastösen, die magnetisierbaren Partikel enthaltenden Materials fließt entlang des Flächenabschnitts, wobei die Partikel aus dem pastösen Material entfernt werden,
- die Vorrichtung wird in den zweiten Arbeitszustand gebracht, wobei die magnetisierbaren Partikel wieder freigegeben werden,
- die Partikel werden an der Austrittsöffnung gesammelt und gewogen,
- die Dichte der Partikel wird rechnerisch bestimmt.

Im folgenden wird die Erfindung anhand einer Ausführungsbeispiele darstellenden Zeichnung näher erläutert. Es zeigen:
- Fig. 1a,b: eine Vorrichtung zur Bestimmung der Dichte magnetisierbarer Partikel in einem pastösen Material in Front- und Seitenansicht,
- Fig. 2a-c: die Verfahrensschritte des erfindungsgemäßen Verfahrens zur Bestimmung der Dichte magnetisierbarer Partikel in einem pastösen Material unter Verwendung der Vorrichtung aus Fig. 1 in schematischer Ansicht,
- Fig. 3a-c: die Verfahrensschritte des erfindungsgemäßen Verfahrens unter Verwendung einer zu Fig. 2 alternativ ausgeführten Vorrichtung in schematischer Ansicht,
- Fig. 4a,b: die Vorrichtung der Fig. 1 in einer weiteren Ausführung in Front- und Seitenansicht,
- Fig. 5a,b: die Magneteinheit der Vorrichtung aus Fig. 3 in schematischer Seiten- und Frontansicht,
- Fig. 6: eine Darstellung des Feldlinienverlaufs der in Multipolanordnung vorliegenden Permanentmagnete zweier gegenüber liegender Magneteinheiten und
- Fig. 7a-c: die erfindungsgemäße Vorrichtung in einer weiteren besonders einfach zu realisierenden Ausführung in schematischer Ansicht.

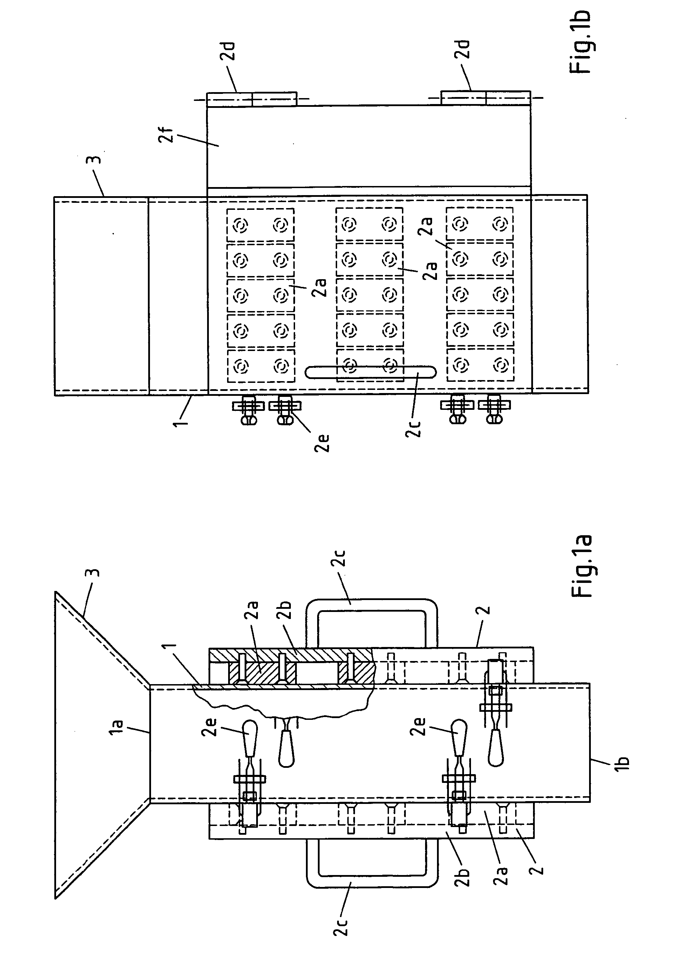

Die in Fig. 1 dargestellte Vorrichtung zur Bestimmung der Dichte magnetisierbarer Partikel in einem pastösen Material umfasst als Grundkomponenten einen Flächenabschnitt und zwei Magneteinheiten 2.

Der Flächenabschnitt ist als Kanalabschnitt 1 mit umfangsmäßig geschlossener Seitenwandung, einer Eintrittsöffnung 1a und einer Austrittsöffnung 1b sowie einem über seine Längserstreckung konstanten, rechteckigen Querschnitt ausgebildet. Die Magneteinheiten 2 sind beidseits des Kanalabschnitts 1 einander gegenüberliegend angeordnet. An der Eintrittsöffnung des Kanalabschnitts 1 ist ein Einfülltrichter 3 angeordnet, welcher das Einfüllen des pastösen Materials in die Vorrichtung erleichtert.

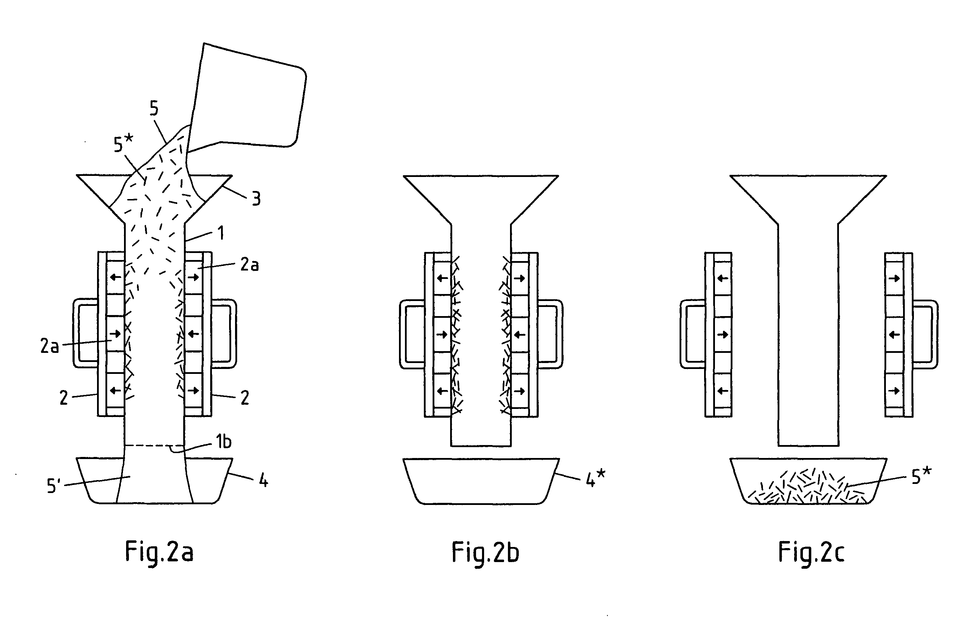

Die Magneteinheiten 2 umfassen jeweils eine Anzahl Permanentmagnete 2a, die jeweils auf einer Grundplatte 2b verschraubt sind. Die Grundplatte besteht vorzugsweise aus einem weichmagnetischen Material, in welchem die Feldlinien zwischen den rückseitigen Polen der Permanentmagnete 2a verlaufen. Die Grundplatte dient somit der Rückleitung des magnetischen Flusses. Die Permanentmagnete 2a sind bezüglich ihrer Polarität derart angeordnet, dass sich jeweils gleiche magnetische Pole gegenüberliegen (vgl. Pfeile in Fig. 2). Die Magneteinheiten 2a sind jeweils über einen Schwenkmechanismus 2f mit Scharnieren 2d mit dem Kanalabschnitt 1 gelenkig verbunden.

Vorliegend befindet sich die Vorrichtung im ersten Arbeitszustand, in dem die Magneteinheiten an der Seitenwandung des Kanalabschnittes 1 anliegen, so dass das von ihnen erzeugte Magnetfeld in den Kanalabschnitt 1 einwirkt. Zur Fixierung der Position der Magneteinheiten und damit zur Sicherung des ersten Arbeitszustandes der Vorrichtung sind die Magneteinheiten über Klemmen 2e mit dem Kanalabschnitt 1 verbunden. Zur Einstellung des zweiten Arbeitszustandes werden die Klemmen 2e gelöst und die Magneteinheiten 2 jeweils von dem Kanalabschnitt 1 weggeschwenkt, was durch entsprechende Schwenkgriffe 2c erleichtert wird.

In den Fig. 2a bis 2c sind die einzelnen Schritte des erfindungsgemäßen Verfahrens zur Bestimmung der Dichte magnetisierbarer Partikel in einem pastösen Material unter Verwendung der Vorrichtung aus Fig. 1 in schematischer Ansicht dargestellt.

Zunächst wird die Vorrichtung in einen ersten Arbeitszustand, in dem das von den beiden Magneteinheiten 2 erzeugte Magnetfeld in den Kanalabschnitt hineinwirkt, gebracht. Dabei kann dies, wie oben beschrieben, über eine Schwenkbewegung erfolgen. Ebenso möglich ist auch ein lineares Heranführen der Magneteinheiten 2. Sodann wird ein definiertes Volumen, beispielsweise 10 Liter, eines pastösen, magnetisierbare Partikel enthaltenden Materials - vorliegend ein Stahlfasern 5* enthaltender, noch fließfähiger Frischbeton 5 - aus einem Vorratsbehälter in den Einfülltrichter 3 der Vorrichtung gegossen. Von dort fließt der Beton 5 in den Kanalabschnitt 1 und durch diesen hindurch. Auf die in dem durch den Kanalabschnitt 1 durchfließenden Beton 5 enthaltenen Stahlfasern 5* üben die in Multipolanordnung vorliegenden Permanentmagnete 2a der Magneteinheiten 2 jeweils eine starke anziehende Kraft aus, so dass die Stahlfasern 5* aus dem Beton 5 entfernt werden, indem sie sich zu den Seitenflächen des Kanalabschnitts 1 hinbewegen und letztlich an der Wandung anhaften, während der von den Stahlfasern 5* vollständig befreite Beton 5' aus der unten liegenden Austrittsöffnung 1b des Kanalabschnittes 1 wieder austritt und in ein Sammelbecken 4 fließt (Fig. 2a). Die erwähnte Multipolanordnung der Permanentmagneten 2a hat den Vorteil, dass somit eine Brückenbildung der Stahlfasern 5* von der einen Seitenwandung zur gegenüber liegenden, was zu einer erheblichen Behinderung des Betonflusses durch den Kanalabschnitt führen würde, vermieden wird.

Wie in Fig. 2b dargestellt, befindet sich die Vorrichtung weiterhin im ersten Arbeitszustand und es haften nun sämtliche Stahlfasern 5* an den den beiden Magneteinheiten 5 jeweils zugewandten Innenseiten des Kanalabschnitts 1 an. Unterhalb der Vorrichtung wird nun ein weiteres Sammelbecken 4* zum Auffangen der Stahlfasern 5* in Position gebracht. Ebenso ist es möglich, die problemlos portabel ausführbare Vorrichtung selbst zu bewegen und über einem geeigneten Sammelbecken zu positionieren.

Anschließend wird nun, wie in Fig. 2c dargestellt, die Vorrichtung - beispielsweise durch Verschwenken der Magneteinheiten 2 - in den zweiten Arbeitszustand gebracht. Dies hat zur Folge, dass die Stahlfasern 5* sich nicht mehr im Wirkungsbereich des Magnetfeldes der Permanentmagneten 2a befinden, folglich keiner anziehenden Kraft, welche sie an der Wandung des Kanalabschnitts 1 fixiert, mehr unterliegen und somit infolge ihrer Gewichtskraft nach unten in das Sammelbecken 4* fallen. Zur Bestimmung der Faserdichte in dem untersuchten Betonvolumen werden die aufgesammelten Fasern im Folgenden gewogen und die Dichte der Partikel anschließend rechnerisch bestimmt.

Als Problem beim Betrieb der Vorrichtung erweist sich regelmäßig, dass in den im ersten Arbeitszustand der Vorrichtung an der Seitenwand des Kanalabschnitts 1 fixierten Stahlfasern 5* weitere im Beton enthaltene Materialien, wie Sand oder andere Zuschlagstoffe, als Verunreinigungen haften bleiben, welche bei der anschließenden Gewichtsmessung der Stahlfasern 5* das Messergebnis verfälschen würden. Um diesem Problem zu begegnen, wird vorzugsweise ein weiterer Prozessschritt zur Abreinigung der Fasern vor Einstellung des zweiten Arbeitszustandes der Vorrichtung, d.h. vor Entfernung der Magneteinheiten 2a, ausgeführt. Dieser kann in einem einfachen Durchspülen des Kanalabschnittes 1 mit einer Reinigungsflüssigkeit, vorzugsweise klarem Wasser, bestehen. Weiterhin ist möglich, die noch verunreinigten Fasern ein weiteres mal durch die Vorrichtung zu leiten. Eine weitere besonders vorteilhafte und bei der Verwendung von Elektromagneten sehr einfach zu realisierende Maßnahme besteht in der Umorientierung des von der Magneteinheit erzeugten Magnetfeldes, was zu einer Drehung der Fasern und damit zu einer Freigabe der anhaftenden Verunreinigungen führt.

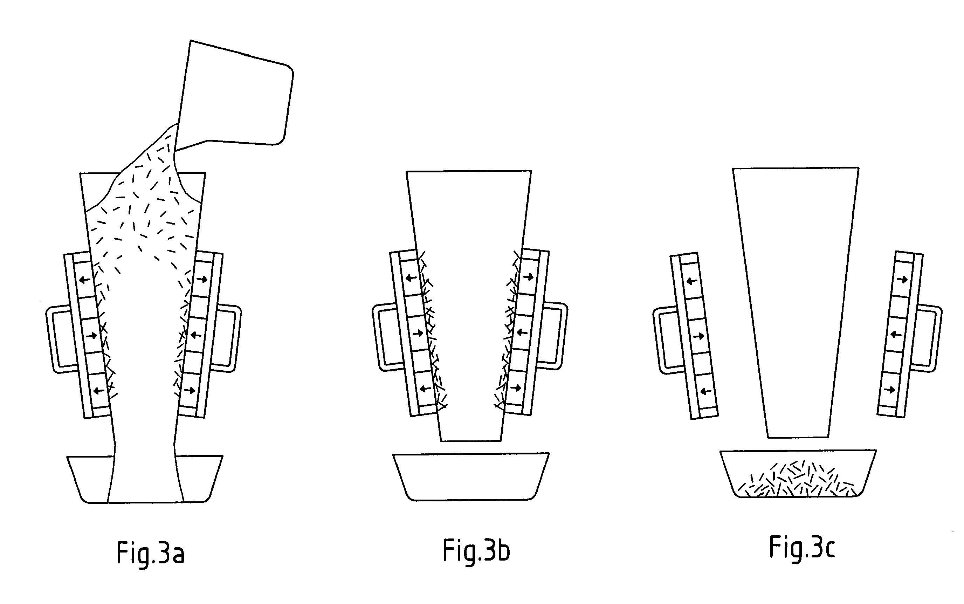

In Fig. 3 sind die Verfahrensschritte des erfindungsgemäßen Verfahrens unter Verwendung einer alternativ ausgeführten Vorrichtung dargestellt. Wie klar erkennbar ist, besteht der einzige Unterschied in einer alternativen Ausführung des Kanalabschnitts. Dieser ist vorliegend konisch, d.h. mit sich in Flussrichtung kontinuierlich verringernder Querschnittsfläche ausgebildet. Dies hat den Vorteil, dass aufgrund des großen Eintrittsquerschnittes ein vorgeordneter Einfülltrichter nicht erforderlich ist. Um den in Richtung der Eintrittsöffnung sich vergrößernden Abstand der Magneteinheiten zu kompensieren, sind die oben angeordneten Magnete vorzugsweise stärker ausgelegt, so dass sie weiter in den Kanalabschnitt einwirken können.

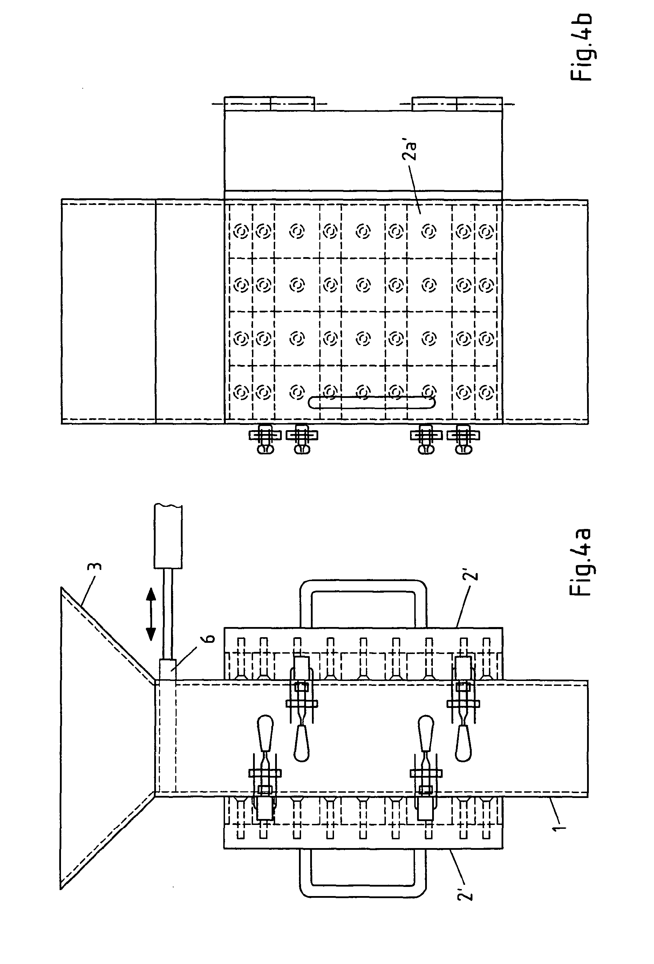

Die in Fig. 4a,b dargestellte Vorrichtung unterscheidet sich von der in Fig. 1 gezeigten durch modifizierte Magneteinheiten 2' sowie durch den Einbau einer als Verschlussschieber 6 ausgebildeten, variablen Verschlusseinrichtung, mithilfe derer ein zunächst in den Einfülltrichter 3 oder in ein der Eintrittsöffnung des Kanalabschnitts vorgeordnetes Speichervolumen (nicht dargestellt) eingefülltes definiertes Betonvolumen zum gewünschten Zeitpunkt in den Kanalabschnitt 1 eingeleitet werden kann.

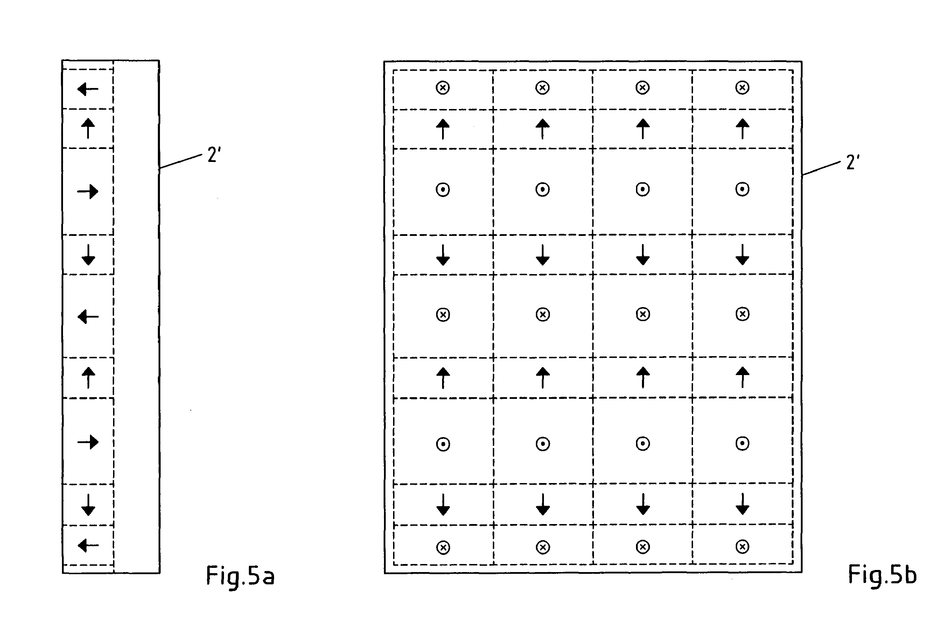

Die in der Vorrichtung der Fig. 4a, b vorgesehene modifizierte Magneteinheit 2' ist in Fig. 5 schematisch dargestellt. Die Magneteinheit 2' umfasst wiederum eine Multipolanordnung von Permanentmagneten. Vorliegend sind diese als Halbach-Array ausgebildet. Eine solche Magnetkonfiguration hat den Vorteil, dass sie nicht auf ein rückseitig angeordnetes weichmagnetisches Material zur Rückleitung des magnetischen Flusses angewiesen ist. Im Falle einer Magnetanordnung als Halbach-Array kann folglich die Grundplatte 2b deutlich dünner ausgeführt und aus einem einfachen nichtmagnetischen Material gefertigt sein. Alternativ kann bei entsprechender Konstruktion auf die Grundplatte ganz verzichtet werden. In jedem Falle ergibt sich durch die Verwendung eines Halbach-Arrays ein signifikantes Potenzial zur Gewichtseinsparung.

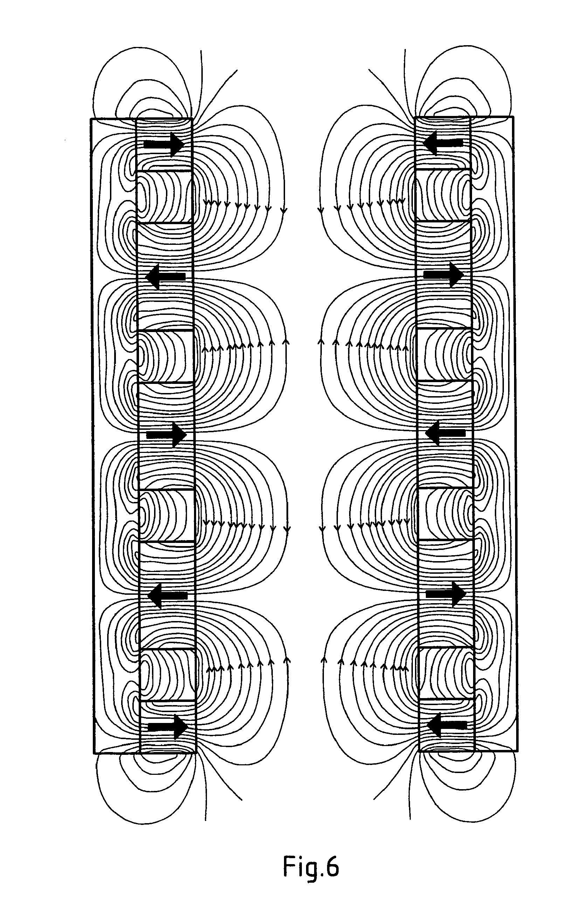

In Fig. 6 schließlich ist der Feldlinienverlauf der in Multipolanordnung vorliegenden Permanentmagnete zweier gegenüber liegender Magneteinheiten dargestellt (vgl. Fig. 2). Dabei wird deutlich, dass die Gefahr einer Brückenbildung durch die magnetisierbaren Partikel zwischen den gegenüber liegenden Magneteinheiten praktisch ausgeschlossen ist. Ferner ist anhand des Feldlinienbildes erkennbar, dass sich die magnetisierbaren Partikel aufgrund der Multipolanordnung gleichmäßig über die gesamte Länge jeweils einer Magneteinheit verteilen, dass es folglich nicht zu einer den Durchflussquerschnitt stark verengenden Klumpenbildung kommen kann.

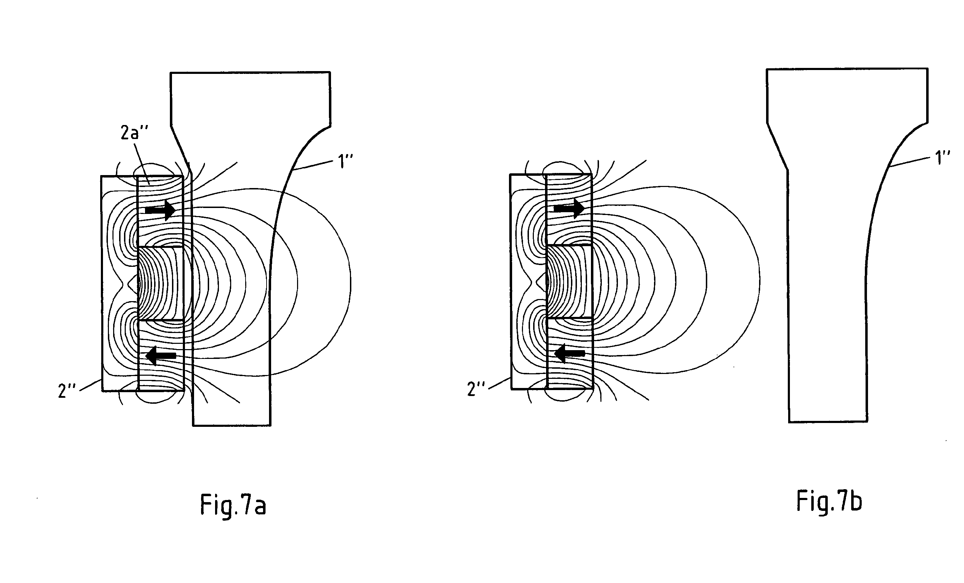

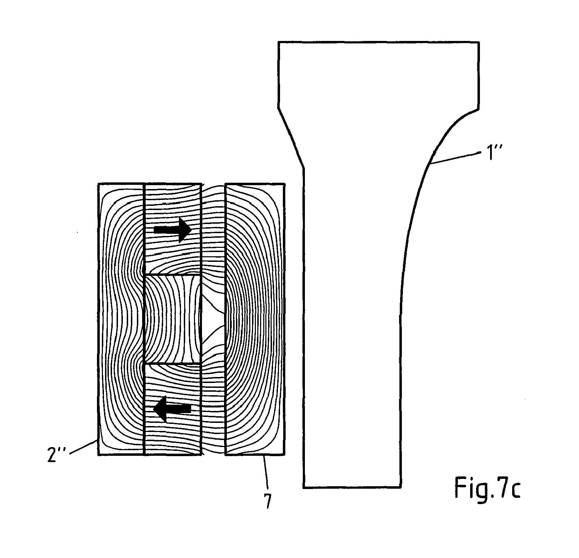

In den Fig. 7a bis c ist schließlich eine besonders einfach zu realisierende, kostengünstig herzustellende und gewichtssparende Variante der erfindungsgemäßen Vorrichtung dargestellt. Sie unterscheidet sich von den bisher dargestellten dadurch, dass sie lediglich eine Magneteinheit 2'' mit Permanentmagneten 2a'' in Multipolanordnung umfasst. Der Kanalabschnitt 1'' ist mit sich in Richtung der Austrittsöffnung 1b'' verjüngendem Querschnitt ausgebildet, so dass einerseits eine Verstopfung des Kanalabschnitts 1" durch das pastöse Material verhindert wird und andererseits sämtliche magnetisierbaren Partikel im ersten Arbeitszustand der Vorrichtung durch die auf sie von den Permanentmagneten 2a" ausgeübte Anziehungskraft aus dem pastösen Material entfernt werden können.

In Fig. 7a ist die Vorrichtung im ersten Arbeitszustand dargestellt. Die Magneteinheit 2a'' ist dabei unmittelbar an der Wandung des Kanalabschnitts 1" positioniert, so dass das von den Magneten 2a'' erzeugte Feld sich praktisch vollständig in den Kanalabschnitt hinein erstreckt und somit eine anziehende Kraft auf die in einem durch den Kanalabschnitt 1'' durchfließenden pastösen Material enthaltenen magnetisierbaren Partikel ausübt.

In Fig. 7b ist die Vorrichtung im zweiten Arbeitszustand dargestellt. Hierbei ist die Magneteinheit 2'' soweit von dem Kanalabschnitt entfernt worden, dass das Magnetfeld nicht mehr in den Kanalabschnitt 1" hineinreicht, so dass die an der Wandung des Kanalabschnittes 1'' anhaftenden Partikel nach unten aus dem Kanalabschnitt 2a'' herausfallen und gesammelt werden können.

In Fig. 7c ist die Vorrichtung ebenfalls im zweiten Arbeitszustand dargestellt. Hierbei ist die Magneteinheit 2" jedoch nicht soweit von dem Kanalabschnitt 1" entfernt worden, dass das Magnetfeld nicht mehr in den Kanalabschnitt 1'' hineinreicht. Stattdessen ist in den schmalen Zwischenraum zwischen Kanalabschnitt 1" und Magneteinheit 2" eine Abschirmplatte 7 eingeführt, die vorzugsweise aus einem weichmagnetischen Werkstoff besteht und das Magnetfeld annähernd vollständig gegenüber dem Kanalabschnitt 1" abschirmt. Folglich kann auch auf diese Art der zweite Arbeitszustand der Vorrichtung, in dem die die magnetisierbaren Partikel wieder freigegeben werden, eingestellt werden.

Die vorstehend beschriebenen Vorrichtungen, insbesondere die mit nur einer Magneteinheit versehene Vorrichtung (s. Fig. 7a bis c), können problemlos portabel ausgeführt werden, so dass sie beispielsweise auf einer Baustelle an verschiedenen Stellen schnell zum Einsatz kommen können. Dazu können die Schwenkgriffe 2c (s. Fig. 1a) als Tragegriffe eingesetzt werden. Falls beispielsweise eine große Fläche, z.B. ein Gebäudefundament mit mehreren Betonchargen gegossen werden soll, so können die Chargen mit der erfindungsgemäßen Vorrichtung einzeln untersucht werden, wodurch die makroskopische Verteilung der Stahlfasern über die gesamte gegossene Fläche bestimmt werden kann.

## Patentansprüche

1. Vorrichtung zur Bestimmung der Dichte magnetisierbarer Partikel in einem pastösen Material (5) mit einem Flächenabschnitt (1), entlang dessen das pastöse (5) Material fließt und mit wenigstens einer ein Magnetfeld erzeugenden Magneteinheit (2), wobei das Magnetfeld der wenigstens einen Magneteinheit (2) in einem ersten Arbeitszustand der Vorrichtung eine anziehende Kraft auf die magnetisierbaren Partikel (5*) in dem entlang des Flächenabschnitts (1) fließenden pastösen Material (5) derart ausübt, dass die in dem pastösen Material (5) enthaltenen magnetisierbaren Partikel (5*) aus dem pastösen Material (5) entfernt werden, und wobei das Magnetfeld der wenigstens einen Magneteinheit (2) in einem zweiten Arbeitszustand der Vorrichtung die magnetisierbaren Partikel (5*) wieder freigibt.

2. Vorrichtung nach Anspruch 1,
**dadurch gekennzeichnet, dass**
der Flächenabschnitt (1) als Kanalabschnitt (1) mit einer Seitenwandung sowie einer stirnseitigen Eintrittsöffnung (1a) und einer stirnseitigen Austrittsöffnung (1b) ausgebildet ist.

3. Vorrichtung nach Anspruch 2,
**dadurch gekennzeichnet, dass**
der Kanalabschnitt (1) eine umfangsmäßig geschlossene Seitenwandung aufweist.

4. Vorrichtung nach Anspruch 3,
**dadurch gekennzeichnet, dass**
der Kanalabschnitt (1) im wesentlichen vertikal mit oben liegender Eintrittsöffnung (1a) angeordnet ist.

5. Vorrichtung nach einem der Ansprüche 2 bis 4 ,
**dadurch gekennzeichnet, dass**
der Kanalabschnitt (1) einen rechteckigen, insbesondere quadratischen Querschnitt aufweist.

6. Vorrichtung nach einem der Ansprüche 2 bis 5,
**dadurch gekennzeichnet, dass**
der Kanalabschnitt (1) eine über die Längserstreckung des Kanalabschnitts (1) konstante Querschnittsfläche aufweist.

7. Vorrichtung nach einem der Ansprüche 2 bis 6,
**dadurch gekennzeichnet, dass**
sich die Querschnittsfläche des Kanalabschnitts (1) entlang der Längserstreckung des Kanalabschnitts (1) verändert.

8. Vorrichtung nach Anspruch 7,
**dadurch gekennzeichnet, dass**
sich die Querschnittsfläche des Kanalabschnitts (1) von der Eintrittsöffnung (1a) des Kanalabschnitts (1) in Richtung der Austrittsöffnung (1b) des Kanalabschnitts (1) verringert.

9. Vorrichtung nach einem der Ansprüche 2 bis 8,
**dadurch gekennzeichnet, dass**
die Eintrittsöffnung (1a) mit einem Einfülltrichter (3) versehen ist.

10. Vorrichtung nach einem der Ansprüche 2 bis 9,
**dadurch gekennzeichnet, dass**
der Eintrittsöffnung (1a) in Flussrichtung des pastösen Materials (5) ein Speichervolumen für das pastöse Material (5) vorgeordnet ist.

11. Vorrichtung nach einem der Ansprüche 2 bis 10,
**dadurch gekennzeichnet, dass**
die Eintrittsöffnung (1a) durch eine variable Verschlusseinrichtung (6) verschließbar ist.

12. Vorrichtung nach einem der Ansprüche 1 bis 11,
**dadurch gekennzeichnet, dass**
die Magneteinheit (2) wenigstens einen Elektromagneten umfasst.

13. Vorrichtung nach einem der Ansprüche 1 bis 12,
**dadurch gekennzeichnet, dass**
die Magneteinheit (2) wenigstens einen Permanentmagneten umfasst, welcher relativ zu dem Flächenabschnitt aus einer ersten Position, in der sich die Vorrichtung im ersten Arbeitszustand befindet, in eine zweite Position, in der sich die Vorrichtung im zweiten Arbeitszustand befindet, und in umgekehrter Richtung bewegbar ist.

14. Vorrichtung nach Anspruch 2 und 13,
**dadurch gekennzeichnet, dass**
die Magneteinheit (2) in der ersten Position mit dem Kanalabschnitt (1) durch Klemmen verbindbar ist.

15. Vorrichtung nach Anspruch 13,
**dadurch gekennzeichnet, dass**
die Vorrichtung eine mit dem Flächenabschnitt (1) fest verbundene Aufnahmeeinrichtung umfasst, in welche der wenigstens eine Permanentmagnet einführbar ist.

16. Vorrichtung nach Anspruch 15,
**dadurch gekennzeichnet, dass**
die Aufnahmevorrichtung mit dem Flächenabschnitt permanent verbunden ist.

17. Vorrichtung nach einem der Ansprüche 1 bis 16,
**dadurch gekennzeichnet, dass**
die Magneteinheit (2) eine Multipol-Anordnung aufweist.

18. Vorrichtung nach Anspruch 17,
**dadurch gekennzeichnet, dass**
die Multipolanordnung der Magneteinheit (2') als Halbach-Array ausgebildet ist.

19. Verfahren zur Bestimmung der Dichte magnetisierbarer Partikel in einem pastösen Material unter Verwendung einer Vorrichtung gemäß den Ansprüchen 1 bis 18, **gekennzeichnet durch** die folgenden Verfahrensschritte:
- die Vorrichtung wird in den ersten Arbeitszustand gebracht
- ein definiertes Volumen des pastösen, die magnetisierbaren Partikel (5*) enthaltenden Materials fließt entlang des Flächenabschnitts, wobei die Partikel (5*) aus dem pastösen Material (5) entfernt werden,
- die Vorrichtung wird in den zweiten Arbeitszustand gebracht, wobei die magnetisierbaren Partikel (5*) wieder freigegeben werden,
- die Partikel (5*) werden gesammelt und gewogen,
- die Dichte der Partikel (5*) wird rechnerisch bestimmt.

20. Verfahren nach Anspruch 19,
**dadurch gekennzeichnet, dass**
als pastöses Material (5) stahlfaserverstärkter Beton verwendet wird.
